Europäisches Patentamt

European Patent Office

Office européen des brevets

(11) Publication number: **0 143 638**
A2

# (12) EUROPEAN PATENT APPLICATION

(21) Application number: **84308196.9**

(22) Date of filing: **27.11.84**

(51) Int. Cl.⁴: **B 29 C 47/00,** B 29 C 47/06, B 29 C 67/20 // A61F2/06 , B29L23:00

(30) Priority: **28.11.83 US 555744**

(43) Date of publication of application: **05.06.85**
**Bulletin 85/23**

(84) Designated Contracting States: **DE FR GB IT**

(71) Applicant: **SHILEY INCORPORATED, 17600 Gilette, Irvine California (US)**

(72) Inventor: **Servas, Francis Martin, 14941 Pinehaven Road, Irvine Orange County, California (US)**
Inventor: **Cottonaro, Cliff Nicholas, 2206 North Hathway, Santa Ana Orange County, California (US)**

(74) Representative: **Graham, Philip Colin Christison et al, Pfizer Limited Ramsgate Road, Sandwich, Kent CT13 9NJ (GB)**

(54) Method for making tubular structures for biomedical applications.

(57) A method for making flexible porous tubular structures useful as synthetic vascular prostheses is disclosed. The number and size of pores can be controlled, and can be varied through the tube wall. The process comprises extruding a polymer solution-salt dispersion through a tube dye in the presence of water, whereby the solvent and salt are extracted into the water leaving the plastic in a sponge-like tubular form.

ACTORUM AG

-1-

P.C. 6748

## METHOD FOR MAKING TUBULAR STRUCTURES
### FOR BIOMEDICAL APPLICATIONS

This invention relates to porous polymer structures. More particularly, it relates to porous synthetic polymer tubular structures useful as vascular prostheses.

Experience has shown that for a synthetic vascular prosthesis to be successful it must possess a number of special chemical and physical properties. It must be non-toxic and chemically stable, and unaffected by prolonged contact with body fluids. It must be flexible, to conform to a desired shape and adjust to body movements; stiff enough to retain its passageway open even when bent; tough enough to hold suturing threads; and porous to permit tissue ingrowth. This combination of requirements has been sought with varying degrees of success in the past by a variety of structures. Widest use has been made of woven and knitted fabric tubes, typically made of polyester fibers. Also used have been porous expanded polytetrafluoroethylene plastic, non-woven felt and paper.

Recently, different types of tubular structures were disclosed in Canadian Patent No. 1,092,303 and British Patent No. 1,552,388. The walls of these tubes are entirely, or in part, composed of inert sponge-like polymer. This spongy polymer has about 10 to about 70% pore volume. The pores are interconnected and about 1 micron to about 1,000 microns in size. This structure is created by dissolving a blood compatible polymer in a volatile solvent to create a viscous solution. A solvent insoluble, but water soluble, salt of a particle size approximately equal to the pore size desired is dispersed in the viscous solution. This viscous dispersion is formed into a desired shape, and dried of solvent. The water soluble salt is then leached out to leave interconnected pores in a polymer matrix. Porosity can be varied throughout wall thickness by laminating layers of different pore size. In some cases, it may be desirable to have one layer of the composite non-porous. It is advantageous to be able to provide a controlled porosity gradient through the vascular prosthesis wall, because tissue ingrowth from the inner or blood side is of a different nature than that ingrowing from the outer or soft tissue side. In general, a smaller inside pore size is desired compared with that of the outside.

A superior way has now been discovered to produce a tubular vascular prosthesis of the type disclosed in the above Canadian and British patents. In this new process, the solvent used is chosen to be not only a good solvent for the polymer, but water miscible and not necessarily especially volatile. With that change, the viscous polymer dispersion with water soluble particles can be extruded through a tube dye into water where both the solvent and particulate matter are simultaneously dissolved away from the water insoluble polymer. Thus, the polymer is coagulated as a tube with open pores in the walls where particles had been. The chosen solvent and the pore forming particles must be relatively non-toxic so that small traces remaining in a prosthesis will not cause adverse reaction in the body.

In this new process, porosity can be made to vary through the tube wall by forming polymer dispersions of particles with different particle size distribution, and co-extruding them in a concentric tube dye as a multilayer tube. There is no need to form separate layers and then be required to laminate those separate parts together as required by the Canadian and British methods.

Therefore, it is an object of this invention to provide a process for making tubular structures of controlled porosity. It is another object to provide a process for forming in one step tubular structures in which porosity can be varied through the tube wall. It is still another object to provide an essentially continuous process for making porous polymer structures.

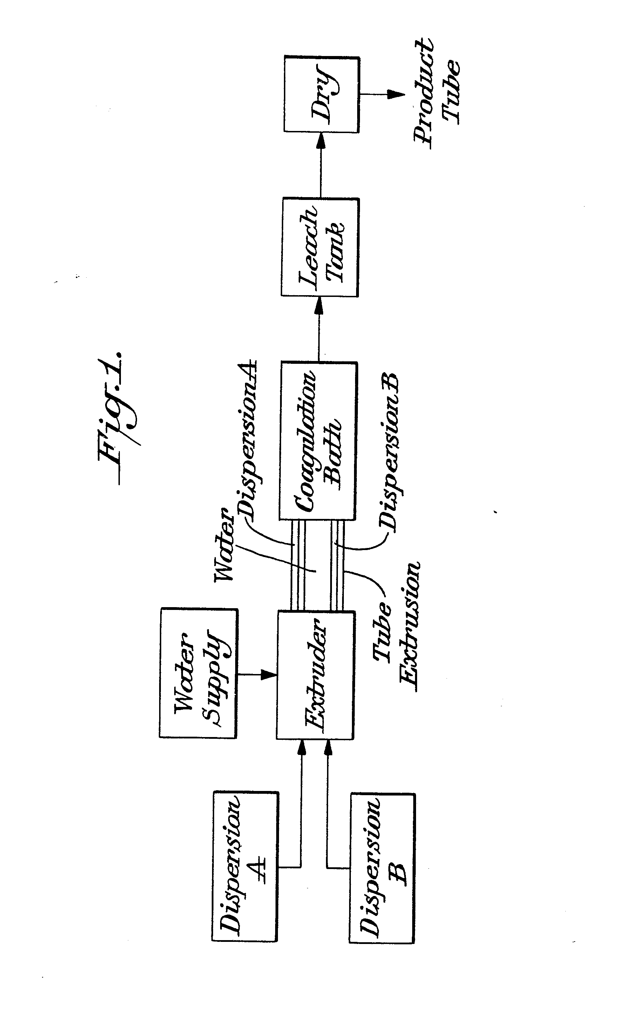

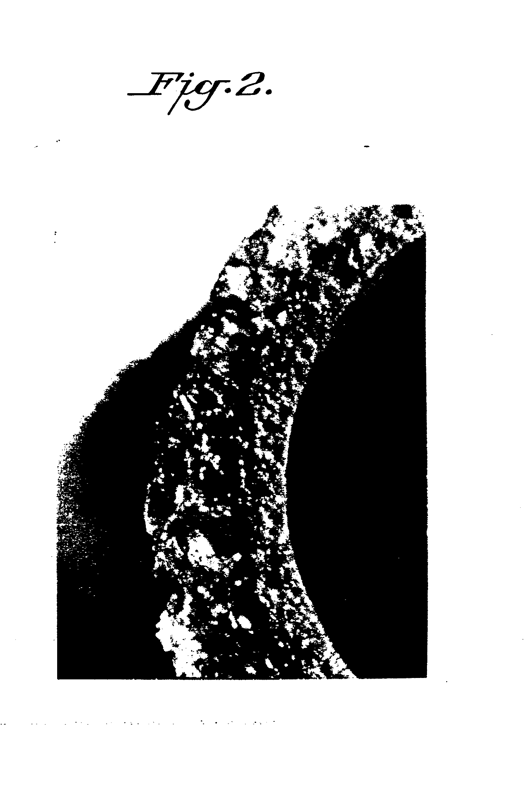

Figure 1 is a schematic diagram of a preferred process according to this invention. Figure 2 is a cross section view magnified 30 times of a dual layer tube as produced by the process of Figure 1.

A particularly useful product made by the process of this invention is a flexible porous tube in the form of a vascular prosthesis in which the average pore size on the inside tube surface is relatively small while the average pore on the outer surface is larger. The process of this invention is well suited to the production of such a variable porosity tube.

Figure 1 is a schematic flow sheet of a preferred process for making a two layer vascular prosthesis. In this process, separate polymer dispersions are formed, one for each of the two different porosity layers. Each dispersion comprises a body-compatible, water insoluble, inherently flexible polymer dissolved in a water miscible solvent and contains a solid phase of a preselected particle size distribution dispersed in it. It has been found that pore size in the final product is approximately equal to particle size of the solid phase used. Therefore, the preselected particle size of solid phase will be approximately that of the desired product pore size. This solid phase must be substantially insoluble in the solvent-polymer solution, but soluble in water.

In the Figure 1 process, Dispersion A, which creates the outer tube layer, will contain larger insoluble particles than will Dispersion B. Consequently, the outer surface of tube wall will have larger pores than the inner surface.

The two viscous dispersions are extruded through a concentric tube dye while water is introduced to the tube interior. The whole is extruded into a water bath. The two polymer dispersions leaving the concentric dye openings merge into a single tube. The high viscosity of these dispersions limits interdiffusion, so a tubular extrusion with concentric layers is formed. This extrusion being surrounded by water, there is a simultaneous extraction of the water soluble ingredients and coagulation of the polymer.

The mechanism of pore formation is not known, but one possibility may be that the solvent is quickly leached into the surrounding water, causing the polymer to coagulate around the more slowly dissolving particles, initially forming a closed structure. Continued leaching by water dissolves away the particles leaving voids in the polymer tube, and producing an open porous sponge-like tube wall. A drying step to remove water completes the basic process. The process can be virtually continuous.

Figure 2 shows in 30 power magnification the dual layer porous structure of a tube made by the process of Figure 1.

The operation of this new process to produce a vascular prosthesis requires that the starting ingredients have special properties. All ingredients must be relatively non-toxic to the human body. The polymer must be water insoluble and stable for long term exposure within the body. Further, the polymer must be substantially soluble in a solvent that is water miscible. The solvent, in addition to being relatively non-toxic and water miscible, must not have such an odor that residual traces in the product would be objectionable.

The solid phase material must be substantially insoluble in the solvent used and in the polymer solvent solution. It must be readily soluble in water and easily produced in a range of desired particle size distributions. An inorganic salt is preferred.

While a number of ingredients may be suitable in this process, the preferred ones are: for polymer, a polyether-urethane, such as those supplied under the names "BIOMER" (Johnson & Johnson Inc.) and "PELLATHANE" (The Upjohn Company); for solvent, dimethylformamide; for solid phase, sodium chloride crystals. Useful concentrations of the polymer in solvent solution have ranged from about 1 to about 50 weight percent. The solid phase salt concentration is conveniently expressed as a weight percent of the polymer. Since each salt particle, or clump of particles, of significant size when dispersed in the polymer-solvent solution will result in an approximately equivalent sized pore, both the number concentration and average size of salt particles must be taken into account when planning a desired tube wall texture. Salt crystals with average particle sizes ranging from about 1 micron to about 250 microns in diameter are useful in concentrations of about 0.01 to about 100 weight percent of the polyurethane. These concentrations create pore volumes of about 1 to 90 percent in the product. Extruded wall thicknesses can be varied from about .010" to about 0.500". Tubes as small as 1mm internal diameter and as large as 50mm internal diameter can be produced.

This process is adaptable to make a wide range of product porosities. A tube may be made from a single polymer dispersion and relatively uniform in properties throughout, or from several dispersions resulting in multiple layers, or even longitudinal stripes and other patterns, of different porosity. It is sometimes useful to have a tube containing a non-porous layer. In that case, one of the polymer feeds to the extruder would contain no insoluble salt particles, and thus coagulate into a layer containing no pores.

It will be appreciated that tubes can be made by this process for many different uses. Also, this process is adaptable to form other shapes than tubes. If desired, the polymer can be formulated to result in a relatively rigid structure, rather than a flexible one. In multi-layer products, not only can the porosity be varied, but different polymers can be used in the different layers. Among products contemplated other than vascular prostheses are drainage tubes, blood filters, cannulae, and trachea prostheses. The process can also make porous plastic forms reinforced with fibers and fillers.

In an alternate process according to this invention, the polymer dispersion can be concentrated, having a highly viscous, putty-like consistancy. Such a dispersion can be formed into a desired shape, and it will hold that shape through subsequent processing steps. The shaped dispersion is extracted with water to simultaneously leach out solvent and solid particles, and to coagulate the polymer into porous form. An advantage of this alternate process is that it can be used to produce intricate shapes impossible to extrude and porous polymer coatings on otherwise solid protheses.

The present invention is illustrated by the following examples. It will however, be understood that the invention is not limited to the specific details and conditions of these examples.

0143638

-8-

### Example 1

A 30;% solution (Solution A) of the polyether-urethane "BIOMER" (Solution Grade) in dimethylformamide (DMF) was mixed with sodium chloride (NaCl) crystals of average size 1 micron in a 1:1 weight ratio of NaCl to polymer to form a viscous dispersion A. Another 30% solution (Solution B) of the same polyether-urethane in DMF was mixed with NaCl crystals of average size 250 micron in a 1:2 weight ratio of NaCl to polymer to form a similar dispersion B. Dispersion A and Dispersion B were extruded through a co-extrusion process as depicted in the process diagram. The inner layer and outer layer were formed from the viscous dispersions A and B, respectively. The extrusion was allowed to proceed in a 23°C coagulation bath followed by a 24 hour soak at 37°C. in the leach tank. The extrusion was allowed to dry at 13°C. The resultant tube exhibited an open porous structure with pore sizes roughly equivalent to the 250 micron crystal for the outer layer and 1 micron for the inner layer.

### Example 2

Dispersions A and B were extruded in the same manner described with the following change in Dispersion B: 10% petroleum jelly was added to the formulation. The tube was treated as before except that a surfactant washing step was added after leaching. The resultant tube exhibited similar porosity to that of Example 1.

### Example 3

The same process was run on each dispersion separately and processed as described in Example 1, using a single tube dye in each case. The resultant single extrusions yielded tubes with pore sizes consistent with the sizes of crystal mixed into the polymer in each case.

P.C. 6748

CLAIMS

1. A method for making plastic articles containing interconnected pores, said method comprising the steps of:

dissolving plastic material in a water miscible solvent;

dispersing a solid phase of preselected particle size distribution into the plastic solution, said solid phase being soluble in water but substantially insoluble in said solvent;

forming the resulting dispersion into a desired shape; and extracting the formed dispersion with water, whereby said solvent and said solid phase are simultaneously extracted into said water phase causing said plastic material to coagulate in porous form into said desired shape, the pore size distribution being determined by the preselected particle size distribution of said solid phase.

2. The method of claim 1 wherein different dispersions of solid phase in plastic solution are made and formed together into said desired shape, whereby layers of plastic material having different pore structure are created in the plastic article produced.

3. The method of claim 1 wherein said dispersion of solid phase in plastic solution is formed together with plastic solution containing no solid phase into said desired shape, whereby a substantially non-porous layer is formed in said product.

4. The method of any preceding claim further comprising the step of drying the extracted porous polymer shape.

5. The method of any preceding claim wherein said forming into a desired shape is accomplished by extruding through a shape-forming dye.

6. The method of claim 5 wherein said desired shape is a tube.

7. The method of claim 6 wherein the extruding is accompanied by injecting water inside the tube form.

8. A tubular prosthesis prepared by the method of claim 6 or claim 7.

*Fig.1.*

1/2

0143638

_Fig.2._